# EUROPÄISCHE PATENTSCHRIFT

(11) **EP 4 311 506 B1**
(45) Veröffentlichungstag und Bekanntmachung des Hinweises auf die Patenterteilung: **09.09.2026**
(21) Anmeldenummer: 23184074.5
(22) Anmeldetag: 07.07.2023
(51) Int. Cl.: A61B 17/15

(54) **CHIRURGISCHES INSTRUMENT UND CHIRURGISCHES INSTRUMENTENSYSTEM**
SURGICAL INSTRUMENT AND SURGICAL INSTRUMENT SYSTEM
INSTRUMENT CHIRURGICAL ET SYSTÈME D'INSTRUMENT CHIRURGICAL

(30) Priorität: 25.07.2022 DE 102022207576
(43) Veröffentlichungstag der Anmeldung: 31.01.2024
(73) Patentinhaber: Aesculap AG, 78532 Tuttlingen (DE)
(72) Erfinder: Firmbach, Franz-Peter, 78576 Emmingen-Liptingen (DE); Stoffels, Lilli, 75365 Calw (DE)
(74) Vertreter: Patentanwälte Ruff, Wilhelm, Beier, Dauster & Partner mbB

(56) Entgegenhaltungen:
- AU-A1- 2010 283 577
- US-A1- 2015 045 801
- US-A1- 2021 000 484

## Beschreibung

Die Erfindung betrifft ein chirurgisches Instrument zur Verwendung bei einer Kniegelenkersatzoperation. Die Erfindung betrifft außerdem ein chirurgisches Instrumentensystem mit einem derartigen chirurgischen Instrument.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs oder/und der Tibia durch künstliche Gelenkflächen einer Kniegelenkprothese ersetzt. Solche Kniegelenkprothesen umfassen üblicherweise eine Femurkomponente und eine Tibiakomponente. Die Femurkomponente wird am distalen Ende des Femurs implantiert. Die Tibiakomponente wird am proximalen Ende der Tibia implantiert.

Vor der Implantation der prothetischen Komponenten werden der distale Femur und die proximale Tibia reseziert. Hierfür bringt der Chirurg unterschiedliche Resektionsschnitte an und trennt Knochen- oder/und Knorpelmaterial von dem jeweiligen Knochen ab. Durch die Resektion wird der jeweilige Knochen in seiner Form an die aufzunehmende prothetische Komponente angepasst.

Die Resektion kann auf der Grundlage unterschiedlicher Konzepte ausgeführt werden. Ein Konzept zielt darauf ab, die Spannungen der Bänder des Knies bei der Gelenkbewegung ausgeglichen zu halten. Hierdurch soll eine bessere Funktion der Kniegelenkprothese gewährleistet werden. Dieses Konzept wird allgemein als "gap balancing" bezeichnet. Bei anderen Konzepten entfernt der Chirurg mittels der Resektion eine bestimmte Menge an Knochen- oder/und Knorpelmaterial. Solche Konzepte werden allgemein als "measured resection" bezeichnet. Die Ausrichtung der Resektionsschnitte in Bezug auf die Anatomie des Patienten bestimmt die spätere Ausrichtung der implantierten Komponenten und folglich auch die Orientierung der prothetischen Gelenkachsen. Die Ausrichtung der Resektionsschnitte ist daher von besonderer Bedeutung.

Bei der Ausrichtung der Resektionsschnitte werden in erster Linie drei Ansätze unterschieden: mechanisch, anatomisch und kinematisch. Bei der mechanischen Ausrichtung wird die proximale Tibia senkrecht zur Längsachse des Tibiaschafts reseziert. Die Resektion des distalen Femurs erfolgt entsprechend hieran angepasst. Erforderlichenfalls werden Bandentlastungen (englisch: ligament releases) durchgeführt. Bei der anatomischen Ausrichtung wird versucht, die Tibia in einem Varus-Winkel von 3° zu resezieren. Die Femurresektion und Bandentlastungen werden mit dem Ziel einer geraden Hüft-Knie-Knöchel-Achse des Beins durchgeführt. Ziel der kinematischen Ausrichtung (englisch: kinematic alignment, nachfolgend abgekürzt als KA) ist es, die künstlichen Gelenkflächen der prothetischen Komponenten auf dem Niveau der präarthritischen, defektfreien natürlichen Gelenkflächen zu implantieren.

Bei einem KA erfolgt die Ausrichtung der Resektionsschnitte oftmals ausgehend von dem distalen Femur. Die Resektion der proximalen Tibia erfolgt hieran angepasst. In diesem Zusammenhang wird auch von einer Übertragung der Ausrichtung oder/und Schnitte gesprochen. Zu diesem Zweck sind spezielle chirurgische Instrumente bekannt, die auch als tibiales Ausricht- oder/und Übertragungswerkzeug (tibial cut alignment guide) bezeichnet werden. Solche Instrumente erlauben eine Übertragung der Ausrichtung der femoralen Resektionsschnitte auf die Tibia. Die Übertragung erfolgt üblicherweise nach einer wenigstens distalen Resektion des Femurs, bei welcher die distalen Kondylen abgetrennt werden. Die Übertragung kann in Extension oder Flexion erfolgen. Bei einer Variante der KA wird zunächst der distale Femur vollständig präpariert (all femur first). Dabei werden die am distalen Femur vorzunehmenden Resektionsschnitte an der Anatomie des Patienten ausgerichtet und am distalen Femur angebracht. Anschließend wird ein femorales Probekondylenimplantat an dem distalen Femur angebracht. Das bereits nach der Anatomie des Patienten ausgerichtete femorale Probekondylenimplantat wird dann als Referenzkomponente herangezogen, deren Ausrichtung mittels des chirurgischen Instruments oder Übertragungswerkzeugs auf die proximale Tibia übertragen wird, um die daran vorzunehmenden Resektionsschnitte auszurichten und anzubringen.

Solche chirurgischen Instrumente weisen üblicherweise mehrere Führungen oder/und Lager auf, die verschiedene Komponenten des jeweiligen chirurgischen Instruments verstellbeweglich miteinander verbinden, um eine Anpassung an gegebene anatomische Besonderheiten des Patienten zu ermöglichen. Derartige Führungen oder Lager sind bei der Verwendung des chirurgischen Instruments während der Kniegelenkersatzoperation aufwändig zu justieren. Außerdem summieren sich vorhandene Führungs- oder Lagerspiele in der Art einer Toleranzkette auf, was einen präzisen Ausrichtungstransfers auf die proximale Tibia abträglich ist.

Dokument US 2015/045801 A1 offenbart ein chirurgisches Instrument zur Verwendung bei einer Kniegelenkersatzoperation.

Aufgabe der Erfindung ist es, ein chirurgisches Instrument sowie ein chirurgisches Instrumentensystem bereitzustellen, die Vorteile gegenüber herkömmlichen chirurgischen Instrumenten und chirurgischen Instrumentensystemen bieten und die insbesondere eine besonders präzise Ausrichtung der tibiaseitig vorzunehmenden Resektionsschnitte ausgehend von einer femurseitig fixierten Referenzkomponente ermöglichen.

Diese Aufgabe wird durch das Bereitstellen eines chirurgischen Instruments mit den Merkmalen des Anspruchs 1 sowie eines chirurgisches Instrumentensystem mit den Merkmalen des Anspruchs 14 gelöst. Bevorzugte Ausführungsformen sind Gegenstand der abhängigen Patentansprüche.

Das erfindungsgemäße chirurgische Instrument dient zur Verwendung bei einer Kniegelenkersatzoperation und weist auf: eine erste Befestigungseinrichtung, welche zur lösbaren Befestigung an einer an einem distalen Femur angebrachten Referenzkomponente eingerichtet ist, eine zweite Befestigungseinrichtung, welche distal von der ersten Befestigungseinrichtung beabstandet und zur lösbaren Befestigung an einem Tibiaschnittblock zur Schnittführung an einer proximalen Tibia eingerichtet ist, eine Führungseinrichtung, welche einenends mit der ersten Befestigungseinrichtung und anderenends mit der zweiten Befestigungseinrichtung verbunden ist und mittels welcher die zweite Befestigungseinrichtung relativ zu der ersten Befestigungseinrichtung in einer sagittalen Führungsebene geführt schwenkbeweglich ist, und einen Ausrichtstab, der zur Ausrichtung an einer anterioren Kante der Tibia eingerichtet ist, wobei der Ausrichtstab mit der zweiten Befestigungseinrichtung lösbar starr, insbesondere dreh- oder/und schwenkstarr, verbunden ist. Das erfindungsgemäße chirurgische Instrument weist eine besonders geringe Anzahl an Führungen oder Lagern auf, welche die Komponenten des chirurgischen Instruments verstellbeweglich miteinander verbinden. Insbesondere ist bei dem chirurgischen Instrument gemäß der Erfindung auf eine schwenkbewegliche Lagerung des Ausrichtstabs an der zweiten Befestigungseinrichtung verzichtet. Das chirurgische Instrument ist folglich besonders robust und verwindungssteif. Dies ermöglicht einen besonders präzisen Transfer der Ausrichtung der Referenzkomponente auf den Tibiaschnittblock. In der Verwendung des chirurgischen Instruments ist die erste Befestigungseinrichtung lösbar an der femurseitig angebrachten Referenzkomponente befestigt und der Tibiaschnittblock ist lösbar an der zweiten Befestigungseinrichtung befestigt. Die Referenzkomponente ist vorzugsweise ein femorales Probekondylenimplantat. In diesem Fall eignet sich das chirurgische Instrument besonders für eine Verwendung bei der all femur first Variante des KA. Alternativ kann es sich bei der Referenzkomponente um einen distalen Femurschnittblock zur distalen Schnittführung an dem Femur oder dergleichen handeln. Weder die Referenzkomponente noch der Tibiaschnittblock sind Bestandteil des erfindungsgemäßen chirurgischen Instruments. Die Führungseinrichtung erlaubt eine geführte Schwenkbewegung der zweiten Befestigungseinrichtung relativ zu der ersten Befestigungseinrichtung. In der Verwendung des chirurgischen Instruments erlaubt die Führungseinrichtung folglich eine dementsprechend geführte Relativbewegung des Tibiaschnittsblocks gegenüber der femurseitigen Referenzkomponente. Hierdurch kann die Neigung des Tibiaschnittblocks in der Führungsebene eingestellt werden. Die Führungsebene ist sagittal ausgerichtet und folglich anteroposterior sowie proximodistal erstreckt. Vorzugsweise schneidet eine Schwenkachse, um welche die beiden Befestigungseinrichtungen relativ zueinander mittels der Führungseinrichtung schwenkbeweglich geführt sind, eine mechanische Tibiaachse in der sagittalen Führungsebene. Die besagte Neigung wird auch als posteriorer oder anteriorer Slope bezeichnet. Die Einstellbarkeit der Neigung des Tibiaschnittblocks - nachfolgend kurz Slope - erlaubt zum einen eine Anpassung an präoperativ festgelegte Parameter. Zum anderen kann eine tatsächlich vorherrschende Flexions- oder Extensionsstellung des Beins durch die Beweglichkeit der Führungseinrichtung berücksichtigt und ausgeglichen werden. Hierdurch lässt sich sicherstellen, dass eine Einstellung des Slope nicht gleichzeitig zu einer ungewollten Veränderung einer proximodistalen Position des Tibiaschnittblocks und damit einer sogenannten Tibiaschnitthöhe führt.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet.

In Ausgestaltung der Erfindung weist die zweite Befestigungseinrichtung einen Aufnahmeabschnitt auf, in welchem ein Befestigungsabschnitt des Ausrichtstabs entlang einer Aufnahmerichtung aufgenommen ist. Dies erlaubt auf besonders einfach realisierbare Weise die starre lösbare Verbindung des Ausrichtstabs mit der zweiten Befestigungseinrichtung. Der Aufnahmeabschnitt und der Befestigungsabschnitt können in der Art einer engen Spielpassung oder einer Übergangspassung aufeinander maßlich abgestimmt sein, um die Montierbarkeit und Demontierbarkeit des Ausrichtstabs zu gewährleisten.

In weiterer Ausgestaltung der Erfindung weist der Befestigungsabschnitt des Ausrichtstabs wenigstens in einem Teilabschnitt einen unrunden, bevorzugt eckigen, besonders bevorzugt rechteckigen, Querschnitt auf, wobei der Aufnahmeabschnitt der zweiten Befestigungseinrichtung eine Formgebung aufweist, die zur lösbaren starren Verbindung des Ausrichtstabs und der zweiten Befestigungseinrichtung komplementär zum Befestigungsabschnitt, insbesondere wenigstens zu dem Teilabschnitt, des Ausrichtstabs ausgebildet ist. Zwischen Aufnahmeabschnitt und Befestigungsabschnitt besteht somit ein Formschluss, der ein Verdrehen des montierten Ausrichtstabs um eine Längsachse des Befestigungsabschnitts relativ zur zweiten Befestigungseinrichtung verhindert.

In weiterer Ausgestaltung der Erfindung ist der unrunde Querschnitt drehsymmetrisch, insbesondere punktsymmetrisch, ausgebildet, so dass der Ausrichtstab zur lösbaren starren Verbindung mit der zweiten Befestigungseinrichtung in wenigstens zwei Winkelpositionen relativ zur zweiten Befestigungseinrichtung in deren Aufnahmeabschnitt aufnehmbar ist. Der Ausrichtstab kann also in mehreren verschiedenen Winkelpositionen relativ zur zweiten Befestigungseinrichtung montiert werden. Vorzugsweise kann der Ausrichtstab in zwei gegeneinander um 180° verdrehten Winkelpositionen relativ zur zweiten Befestigungseinrichtung montiert werden. Somit kann ein und derselbe Ausrichtstab, insbesondere wenn er im Ganzen eine rotationsunsymmetrische Formgebung aufweist, sowohl bei einer Kniegelenkersatzoperation am rechten wie auch am linken Bein verwendet werden.

In weiterer Ausgestaltung der Erfindung ist der Ausrichtstab S-förmig gebogen. Ein derartiger Ausrichtstab folgt in vorteilhafter Weise der Anatomie der anterioren Kante der Tibia, so dass er sich besonders präzise an der anterioren Kante der Tibia ausrichten lässt.

In weiterer Ausgestaltung der Erfindung weist die zweite Befestigungseinrichtung einen Handhabungsabschnitt auf, dessen Formgebung auf eine Anatomie einer menschlichen Hand abgestimmt ist. Dies verbessert die Bedienbarkeit des chirurgischen Instruments. Insbesondere erlaubt der Handhabungsabschnitt einen besonders sicheren und für den ausführenden Operateur ermüdungsfreien Griff des chirurgischen Instruments.

In weiterer Ausgestaltung der Erfindung umfasst die zweite Befestigungseinrichtung einen, insbesondere plattenförmigen, Grundkörper, an welchem zur Ausbildung des Handhabungsabschnitts eine Ausnehmung vorhanden ist, die zum Aufnehmen wenigstens eines Fingers ausgebildet ist. Ein solcher durch die Ausnehmung gebildeter Handhabungsabschnitt lässt sich besonders einfach herstellen. Zudem ergeben sich ergonomische Vorteile.

In weiterer Ausgestaltung der Erfindung ist eine Anzeigeeinrichtung vorhanden, die zum Anzeigen einer Winkelstellung der zweiten Befestigungseinrichtung in der Führungsebene relativ zur ersten Befestigungseinrichtung eingerichtet ist. Diese Winkelstellung entspricht im montierten Zustand des Ausrichtstabs seiner Winkelstellung relativ zu der ersten Befestigungseinrichtung. Die Anzeigeeinrichtung erlaubt somit ein Ablesen eines momentanen Beugungswinkels des Femurs relativ zur Tibia. Ausgehend von diesem Beugungswinkel lässt sich ferner auf besonders intuitive Weise der Slope einstellen, bevor die proximale Tibia reseziert wird. Vor dem Resezieren der Tibia lässt sich der Ausrichtstab zur besseren Zugänglichkeit vorzugsweise demontieren.

In weiterer Ausgestaltung der Erfindung umfasst die Anzeigeeinrichtung wenigstens eine Skale und wenigstens eine Ablesemarke, wobei die Skale mit der ersten Befestigungseinrichtung und die Ablesemarke mit der Führungseinrichtung verbunden ist oder umgekehrt, und wobei eine Position der Ablesemarke relativ zur Skale die momentane Winkelstellung der zweiten Befestigungseinrichtung in der Führungsebene relativ zur ersten Befestigungseinrichtung repräsentiert. Diese Ausgestaltung ist konstruktiv einfach und erlaubt zudem ein einfaches und schnelles visuelles Erfassen der Winkelstellung.

In weiterer Ausgestaltung der Erfindung weist die Führungseinrichtung wenigstens eine gebogene Führungsstange und eine Führungsaufnahme auf, in welcher die Führungsstange gleitbeweglich geführt aufgenommen ist, wobei die Führungsaufnahme mit der ersten Befestigungseinrichtung und die Führungsstange mit der zweiten Befestigungseinrichtung verbunden ist oder umgekehrt, und wobei die zweite Befestigungseinrichtung mittels der Führungseinrichtung entlang einer kreisbogenförmig längserstreckten Führungsbahn relativ zur ersten Befestigungseinrichtung linearbeweglich geführt ist. Eine derartige Führungseinrichtung kann auch als Kreisbogenführung bezeichnet werden. Sie erlaubt die schwenkbewegliche Verbindung der beiden Befestigungseinrichtungen um eine im Abstand zur Führungseinrichtung angeordnete gedachte (virtuelle) Schwenkachse.

In weiterer Ausgestaltung der Erfindung ist eine erste Linearführung vorhanden, mittels derer die Führungseinrichtung mit der ersten Befestigungseinrichtung verbunden und relativ zu derselben in der Führungsebene entlang einer anteroposterior längserstreckten ersten Führungsbahn geführt linearbeweglich ist. Die erste Linearführung erlaubt es in vorteilhafter Weise, die Schwenkachse der beiden Befestigungseinrichtungen zu verstellen. Damit lässt sich insbesondere die Position der Schwenkachse auf die Anatomie des Patienten abstimmen.

In weiterer Ausgestaltung der Erfindung ist eine zweite Linearführung vorhanden, mittels derer die zweite Befestigungseinrichtung mit der Führungseinrichtung verbunden und relativ zu derselben in der Führungsebene entlang einer anteroposterior längserstreckten zweiten Führungsbahn geführt linearbeweglich ist. Folglich ist eine anteroposteriore Position der zweiten Befestigungseinrichtung an die Anatomie des Patienten anpassbar, insbesondere an die Größe der Tibia. Eine ungewollte Veränderung der anteroposterioren Position der Führungseinrichtung wird hierbei vermieden. Vorgenanntes gilt entsprechend für die Positionierung des Tibiaschnittblocks, wenn dieser an der zweiten Befestigungseinrichtung befestigt ist.

In weiterer Ausgestaltung der Erfindung ist eine Fixiereinrichtung vorhanden und zur Fixierung der Beweglichkeit der Führungseinrichtung eingerichtet. Somit lässt sich der Tibiaschnittblock nach erfolgter Ausrichtung an der Referenzkomponente in seiner Position fixieren. Dies erlaubt eine besonders sichere Führung eines chirurgischen Schnittwerkzeugs beim Anbringen eines Resektionsschnitts an der Tibia.

Die Erfindung betrifft ferner ein chirurgisches Instrumentensystem mit einem erfindungsgemäßen chirurgischen Instrument gemäß der vorhergehenden Beschreibung. Das chirurgische Instrumentensystem umfasst ferner eine Referenzkomponente, die lösbar an der ersten Befestigungseinrichtung des chirurgischen Instruments befestigt ist, und einen Tibiaschnittblock, der lösbar an der zweiten Befestigungseinrichtung des chirurgischen Instruments befestigt ist. Die bereits erläuterten Vorteile des erfindungsgemäßen chirurgischen Instruments übertragen sich mutatis mutandis auch auf das erfindungsgemäße chirurgische Instrumentensystem.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.
- Fig. 1: zeigt eine schematische Perspektivdarstellung einer Ausführungsform eines erfindungsgemäßen chirurgischen Instruments in einer intraoperativen Situation, in welcher das chirurgische Instrument einenends an einem als Referenzkomponente fungierenden femoralen Probekondylenimplantat und anderenends an einem proximalen Tibiaschnittblock lösbar befestigt ist,
- Fig. 2: eine schematische Perspektivdarstellung der in Fig. 1 gezeigten intraoperativen Situation aus einem anderen Blickwinkel,
- Fig. 3: eine schematische Perspektivdarstellung einer demontierten zweiten Befestigungseinrichtung des chirurgischen Instruments nach den Fig. 1 und 2,
- Fig. 4: eine schematische Seitenansicht der zweiten Befestigungseinrichtung nach Fig. 3,
- Fig. 5: eine schematische Draufsicht der zweiten Befestigungseinrichtung nach Fig. 3 und 4,
- Fig. 6: eine schematische Draufsicht eines demontierten Ausrichtstabs des chirurgischen Instruments nach den Fig. 1 und 2,
- Fig. 7: ein Detail einer schematischen Perspektivdarstellung des Ausrichtstabs nach Fig. 6 und
- Fig. 8: ein Detail einer schematischen Perspektivdarstellung des Ausrichtstabs nach den Fig. 6 und 7 aus einem anderen Blickwinkel.

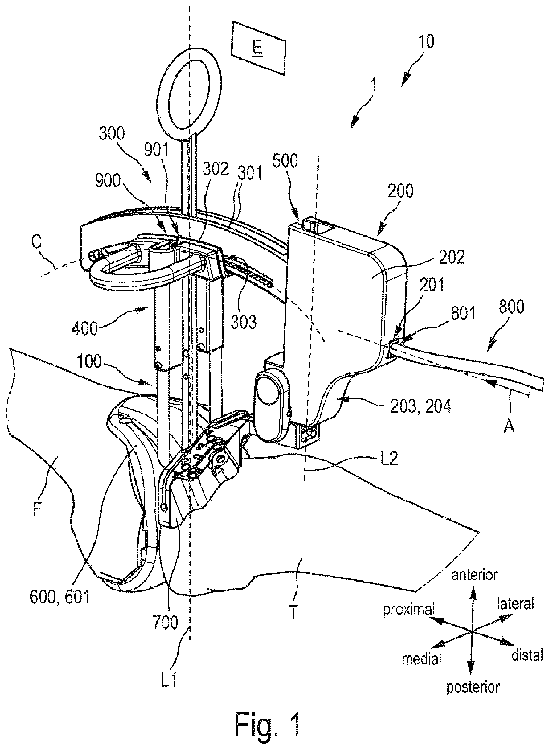

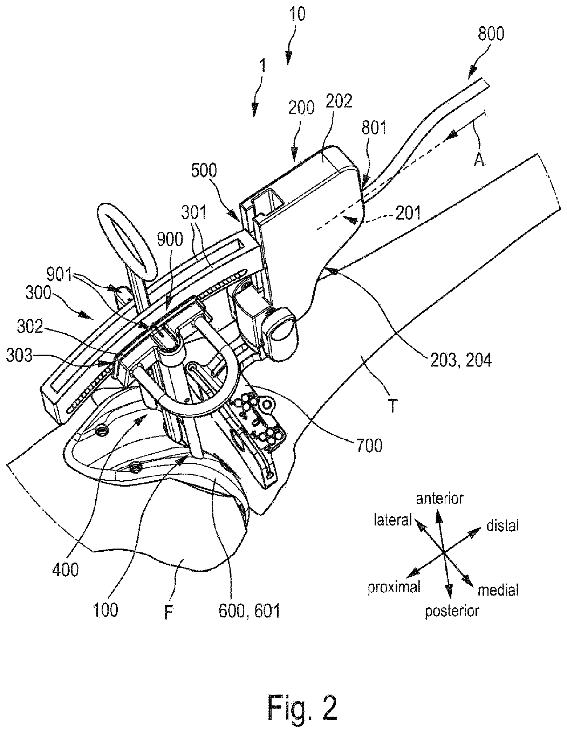

Gemäß den Fig. 1 und 2 ist ein chirurgisches Instrument 1 zur Verwendung bei einer Kniegelenkersatzoperation vorgesehen. Das chirurgische Instrument 1 kann auch als Übertragungswerkzeug oder tibia transfer tool bezeichnet werden. Es dient einer referenzierten Positionierung eines Tibiaschnittblocks 700 an einer proximalen Tibia T. Als Referenz für die Position des Tibiaschnittblocks 700 dient eine bereits an einem distalen Femur F positionierte Referenzkomponente 600. In der in den Fig. 1 und 2 gezeigten intraoperativen Situation ist der distale Femur F bereits vollständig präpariert und mit einem Probekondylenimplantat 601 versehen, welches als Referenzkomponente 600 fungiert. Bevor das femorale Probekondylenimplantat 601 an dem distalen Femur F angeordnet wurde, wurden bereits sämtliche erforderliche Resektionsschnitte an dem Femur F angebracht. Daher bezieht sich die gezeigte intraoperative Situation auf ein sogenanntes all femur first Operationsverfahren, welches sich darin auszeichnet, dass zunächst der distale Femur F vollständig vorbereitet wird, bevor etwaige Maßnahmen an einer proximalen Tibia T unternommen werden. Alternativ zu dem femoralen Probekondylenimplantat 601 kann als Referenzkomponente 600 auch ein Femurschnittblock verwendet werden, falls der Operateur einem anderen als dem all femur first Ansatz folgt.

Die Referenzkomponente 600 und das femorale Probekondylenimplantat 601 sind nicht Bestandteil des chirurgischen Instruments 1. Der Tibiaschnittblock 700, welcher zur Schnittführung an der proximalen Tibia T eingerichtet ist, ist nicht Bestandteil des chirurgischen Instruments 1.

Das chirurgische Instrument 1 bildet gemeinsam mit der Referenzkomponente 600 und mit dem Tibiaschnittblock 700 ein chirurgisches Instrumentensystem 10.

Das chirurgische Instrument 1 umfasst eine erste Befestigungseinrichtung 100, eine zweite Befestigungseinrichtung 200, eine Führungseinrichtung 300 sowie einen Ausrichtstab 800.

Die erste Befestigungseinrichtung 100 ist zur lösbaren Befestigung an der Referenzkomponente 600 eingerichtet. Die zweite Befestigungseinrichtung 200 ist distal von der ersten Befestigungseinrichtung 100 beabstandet. Die zweite Befestigungseinrichtung 200 ist zur lösbaren Befestigung an dem Tibiaschnittblock 700 eingerichtet. In der in den Fig. 1 und 2 dargestellten intraoperativen Situation ist die erste Befestigungseinrichtung 100 lösbar an der Referenzkomponente 600 und die zweite Befestigungseinrichtung 200 lösbar an dem Tibiaschnittblock 700 befestigt.

Die Führungseinrichtung 300 ist einenends mit der ersten Befestigungseinrichtung 100 und andernends mit der zweiten Befestigungseinrichtung 200 verbunden. Die Verbindung der Führungseinrichtung 300 mit der ersten Befestigungseinrichtung 100 sowie - alternativ oder zusätzlich - mit der zweiten Befestigungseinrichtung 200 kann lösbar ausgebildet sein. Vorliegend ist die zweite Befestigungseinrichtung 200 mittels der Führungseinrichtung 300 relativ zu der ersten Befestigungseinrichtung 100 in einer sagittalen Führungsebene E geführt schwenkbeweglich. Somit ermöglicht es die Führungseinrichtung 300, dass die zweite Befestigungseinrichtung 200 relativ zu der ersten Befestigungseinrichtung 100 innerhalb der sagittalen Führungsebene E unter einem Winkel geführt anstellbar ist.

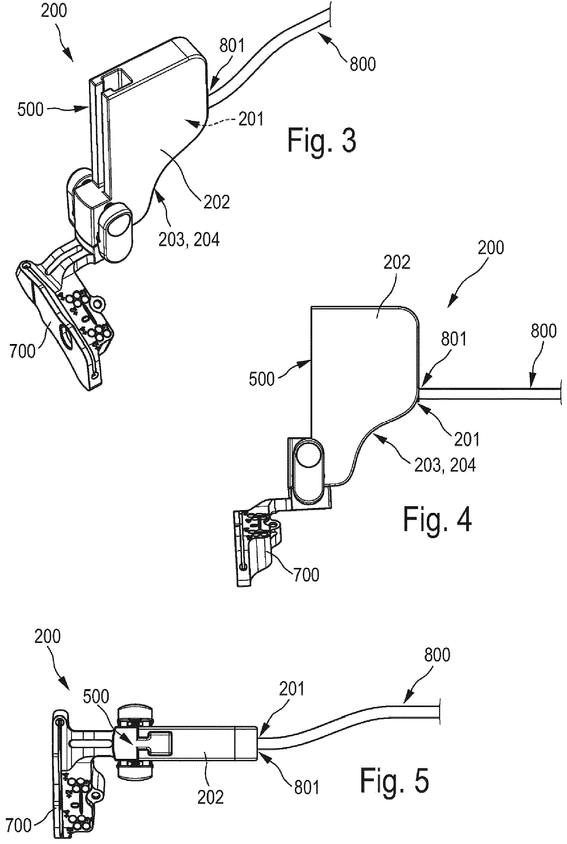

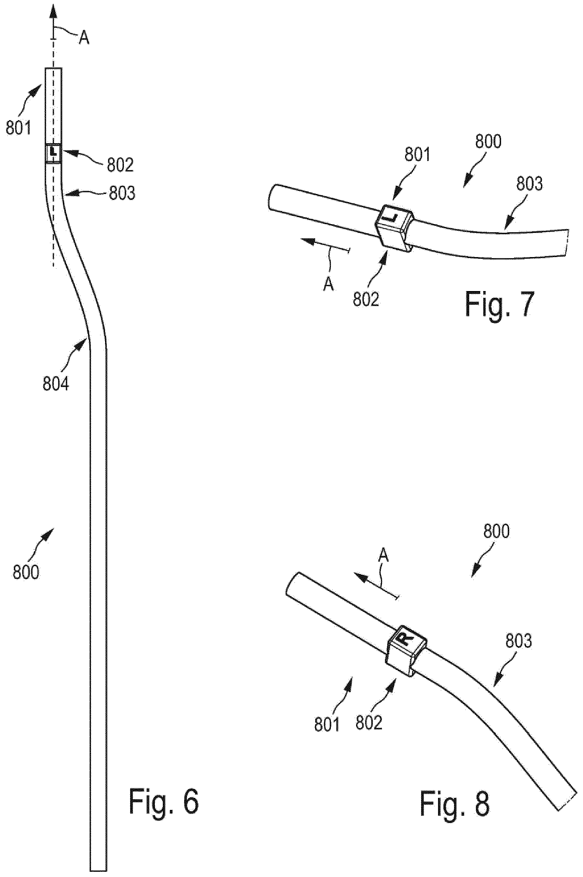

Der Ausrichtstab 800 ist zur Ausrichtung an einer anterioren Kante V der Tibia T eingerichtet. Dabei ist der Ausrichtstab 800 mit der zweiten Befestigungseinrichtung 200 lösbar starr verbunden. In den Fig. 1 und 2 befindet sich der Ausrichtstab 800 in einem montierten Zustand, in welchem er lösbar mit der zweiten Befestigungseinrichtung starr verbunden ist. Vorliegend ist die lösbare Verbindung des Ausrichtstabs 800 mit der zweiten Befestigungseinrichtung 200 dreh- sowie schwenkstarr ausgebildet. Die zweite Befestigungseinrichtung 200, die in den Fig. 3 bis 5 zur besseren Anschaulichkeit gesondert gezeigt ist, weist einen Aufnahmeabschnitt 201 auf. Der Ausrichtstab 800, welcher in den Fig. 6 bis 8 gesondert gezeigt ist, weist einen Befestigungsabschnitt 801 auf. Im montierten Zustand des Ausrichtstabs 800 ist der Befestigungsabschnitt 801 entlang einer Aufnahmerichtung A in dem Aufnahmeabschnitt 201 aufgenommen. Der Ausrichtstab 800 kann in der dem Aufnahmeabschnitt 201 eingesteckt sein. Vorliegend verläuft die Aufnahmerichtung A proximodistal.

Gemäß den Fig. 6 bis 8 umfasst der Befestigungsabschnitt 801 des Ausrichtstabs 800 wenigstens einen Teilabschnitt 802. Der Teilabschnitt 802 weist einen unrunden Querschnitt auf. Vorliegend weist der Teilabschnitt 802 einen eckigen - genauer: einen rechteckigen - Querschnitt auf. Der Querschnitt des Teilabschnitts 801 ist senkrecht zur Aufnahmerichtung A orientiert. Der Aufnahmeabschnitt 201 der zweiten Befestigungseinrichtung 200 weist eine Formgebung auf, die komplementär zu dem Befestigungsabschnitt 801 des Ausrichtstabs 800 ausgebildet ist. Mittels des unrunden Querschnitts und der darauf abgestimmten Formgebung der Befestigungseinrichtung 200 ist die lösbare starre Verbindung des Ausrichtstabs 800 mit der zweiten Befestigungseinrichtung 200 durch Formschluss bewirkt. Der unrunde Querschnitt ist drehsymmetrisch ausgebildet. Vorliegend ist der unrunde Querschnitt punktsymmetrisch ausgebildet. Durch die drehsymmetrische Ausbildung des Querschnitts ist der Ausrichtstab 800 in wenigstens zwei Winkelpositionen relativ zur zweiten Befestigungseinrichtung 200 in dem Aufnahmeabschnitt 201 aufnehmbar. Aufgrund der punktsymmetrischen Ausbildung des unrunden Querschnitts lässt sich der Ausrichtstab 800 vorliegend in zwei relativ zueinander um 180° verdrehten Winkelpositionen montieren.

Der Ausrichtstab 800 ist vorliegend S-förmig gebogen. Der S-förmig gebogene Ausrichtstab 800 weist eine erste Biegung 803 und eine dazu gegenläufige zweite Biegung 804 auf. Durch die beiden Biegungen 803, 804 ist der Ausrichtstab 800 vorliegend gekröpft ausgebildet. Der Ausrichtstab 800 ist sowohl für eine Kniegelenkersatzoperation am rechten wie auch am linken Bein verwendbar, je nachdem, in welcher Winkelposition er an der zweiten Befestigungseinrichtung 200 montiert ist. Welche der Winkelpositionen für eine Kniegelenkersatzoperation am linken Bein bestimmt ist und welche für eine Kniegelenkersatzoperation am rechten Bein bestimmt ist, lässt sich für den Operateur anhand in den Fig. 7 und 8 gezeigten Markierungen - L für links und R für rechts - erkennen. Um das chirurgische Instrument 1 für eine Operation am jeweiligen Bein zu konfigurieren, wird der Ausrichtstab 800 vorliegend so mit der zweiten Befestigungseinrichtung 200 lösbar verbunden, dass die dem jeweiligen Bein zugehörige Markierung nach oben zeigt. Der Ausrichtstab 800 ist zwischen einem proximalen Ende und einem distalen Ende längserstreckt. Der Befestigungsabschnitt 801 des Ausrichtstabs 800 ist an dem proximalen Ende angeordnet.

Gemäß den Fig. 1 bis 5 weist die zweite Befestigungseinrichtung 200 einen Handhabungsabschnitt 203 auf. Eine Formgebung des Handhabungsabschnitts 203 ist auf eine Anatomie einer menschlichen Hand abgestimmt. Dies sorgt für einen sicheren und ermüdungsfreien Griff des Benutzers, beispielsweise beim Montieren oder beim Demontieren des Ausrichtstabs 800. Die zweite Befestigungseinrichtung 200 umfasst einen Grundkörper 202. Vorliegend ist der Grundkörper 202 plattenförmig ausgebildet. An dem Grundkörper 202 ist eine Ausnehmung 204 angeordnet. Durch die Ausnehmung 204 ist der Handhabungsabschnitt 203 ausgebildet. In der Ausnehmung 204 ist wenigstens ein Finger der Hand des Benutzers aufnehmbar. Vorliegend ist die Ausnehmung 204 an einem posterioren und distalen Ende des Grundkörpers 202 angeordnet. Somit bildet die Ausnehmung 204 eine Hinterschneidung aus, die bei der Handhabung des chirurgischen Instruments 1 oder der Befestigungseinrichtung 200 durch Schwerkraftwirkung anteroposterior auf der Hand des Benutzers aufliegt, um einen sicheren Griff zu gewährleisten. Der Grundkörper 202 weist vorliegend eine quaderförmige Formgebung auf.

Vorliegend umfasst das chirurgische Instrument 1 ferner eine Anzeigeeinrichtung 900. Die Anzeigeeinrichtung 900 ist zum Anzeigen einer Winkelstellung der zweiten Befestigungseinrichtung 200 in der Führungsebene E relativ zur ersten Befestigungseinrichtung 100 eingerichtet. Wegen der lösbaren starren Verbindung des Ausrichtstabs 800 mit der zweiten Befestigungseinrichtung 200 entspricht eine in der Führungsebene E gemessene Winkelstellung des Ausrichtstabs 800 relativ zur ersten Befestigungseinrichtung 100 der mittels der Anzeigeeinrichtung 900 angezeigten Winkelstellung. Die Anzeigeeinrichtung 900 umfasst wenigstens eine Ablesemarke 901. Die Anzeigeeinrichtung 900 weist außerdem wenigstens eine Skale auf, die in den Figuren aber nicht erkennbar ist. Die Ablesemarke 901 und die Skale zeigen auf dem Fachmann geläufige Weise in gemeinsamer Wechselwirkung die Winkelstellung an. Die Ablesemarke ist vorliegend mit der ersten Befestigungseinrichtung 100 fest verbunden, wobei die Skale mit der Führungseinrichtung 300 fest verbunden ist. Alternativ hierzu ist es auch denkbar, die Skale oder die Ablesemarke 901 relativ zum sie tragenden Bauteil verstellbeweglich mit dem jeweiligen Bauteil zu verbinden, um die Anzeigeeinrichtung 900 kalibrierbar auszuführen. Eine Position der Ablesemarke 901 relativ zur Skale repräsentiert die momentane in der Führungsebene E gemessene Winkelstellung der zweiten Befestigungseinrichtung 200 relativ zur ersten Befestigungseinrichtung 100. Im montierten Zustand des Ausrichtstabs 800 lässt sich somit auch die momentane Winkelstellung des Ausrichtstabs 800 relativ zur ersten Befestigungseinrichtung 100 ablesen. Die Skale weist eine Teilung mit Winkelinkrementen auf. Die Skale kann in Inkrementen von 1° geteilt sein.

Vorliegend ist die Führungseinrichtung 300 als Kreisbogenführung ausgebildet. Die Führungseinrichtung 300 umfasst wenigstens eine gebogene Führungsstange 301. Vorliegend sind zwei solcher gebogener Führungsstangen 301 vorgesehen. Die Führungsstangen 301 sind parallel zueinander angeordnet. Die Führungsstangen 301 sind an ihren Enden miteinander verbunden. Die Führungseinrichtung 300 umfasst ferner eine Führungsaufnahme 302. In der Führungsaufnahme 302 sind die Führungsstangen 301 gleitbeweglich geführt aufgenommen. Dabei ist die Führungsaufnahme 302 mit der ersten Befestigungseinrichtung 100 verbunden. Die Führungsstangen 301 sind mit der zweiten Befestigungseinrichtung 200 verbunden. Es versteht sich, dass in umgekehrter Weise auch die wenigstens eine Führungsstange 301 mit der ersten Befestigungseinrichtung 100 und die Führungsaufnahme 302 mit der zweiten Befestigungseinrichtung 200 verbunden sein kann, was in den Figuren jedoch nicht gezeigt ist. Mittels der Führungseinrichtung 300 ist die zweite Befestigungseinrichtung 200 entlang einer kreisbogenförmig längserstreckten Führungsbahn C relativ zu der ersten Befestigungseinrichtung 100 linearbeweglich geführt. Durch die linearbewegliche Führung entlang der Kreisbogenbahn C wird die Schwenkbeweglichkeit der ersten Befestigungseinrichtung 100 relativ zur zweiten Befestigungseinrichtung 200 innerhalb der sagittalen Führungsebene E erreicht.

Es ist ferner eine erste Linearführung 400 vorhanden. Mittels der ersten Linearführung 400 ist die Führungseinrichtung 300 mit der ersten Befestigungseinrichtung 100 verbunden. Die Führungseinrichtung 300 ist mittels der ersten Linearführung 400 relativ zur ersten Befestigungseinrichtung 100 in der Führungsebene E entlang einer anteroposterior längserstreckten ersten Führungsbahn L1 geführt linearbeweglich. Das chirurgische Instrument 1 umfasst außerdem eine zweite Linearführung 500. Die zweite Befestigungseinrichtung 200 ist mittels der zweiten Linearführung 500 mit der Führungseinrichtung 300 verbunden. Die zweite Befestigungseinrichtung 200 und die Führungseinrichtung 300 sind relativ zueinander in der Führungsebene E entlang einer anteroposterior längserstreckten zweiten Führungsbahn L2 geführt linearbeweglich. Die beiden Führungsbahnen L1 und L2 befinden sich in derselben Führungsebene E, innerhalb welcher die beiden Befestigungseinrichtungen 100, 200 mittels der Führungseinrichtung 300 relativ zueinander schwenkbeweglich geführt sind. In der in den Fig. 1 und 2 gezeigten intraoperativen Situation sind die beiden Führungsbahnen L1, L2 parallel zueinander erstreckt. Beide Führungsbahnen L1, L2 verlaufen dabei anteroposterior. Werden die beiden Befestigungseinrichtungen 100, 200 relativ zueinander verschwenkt, so können die Führungsbahnen L1 und L2 winkelig gegeneinander angestellt werden. Die erste Linearführung 400 ist in der gezeigten Ausführungsform als Zylinderführung ausgebildet. Die zweite Linearführung 500 ist als Kulissenführung ausgebildet.

Die beiden Befestigungseinrichtungen 100, 200 sind relativ zueinander um eine Schwenkachse schwenkbar. Die Schwenkachse ist eine geometrische oder/und virtuelle Achse. Diese Schwenkachse lässt sich mittels der beiden Linearführungen 400, 500 verlagern. Dabei bleibt die Schwenkachse stets senkrecht zur sagittalen Führungsebene E ausgerichtet. Es versteht sich, dass auch bereits mit einer einzigen Linearführung 400, 500 eine entsprechende Verlagerung der Schwenkachse erreicht werden kann. Die erste Linearführung 400 ist vorliegend einenends der Führungseinrichtung 300 und die zweite Linearführung 500 anderenends der Führungseinrichtung 300 angeordnet.

Die als Kreisbogenführung ausgebildete Führungseinrichtung 300 erlaubt eine geführte Relativbewegung zwischen der zweiten Befestigungseinrichtung 200 und der ersten Befestigungseinrichtung 100. Die zweite Befestigungseinrichtung 200 ist relativ zu der ersten Befestigungseinrichtung 100 entlang der kreisbogenförmig längserstreckten Führungsbahn C geführt linearbeweglich. Dabei führt die zweite Befestigungseinrichtung 200 eine Rotation oder/und Schwenkbewegung um einen Mittelpunkt der Kreisführungsbahn C aus. In dem Mittelpunkt der kreisbogenförmig längserstreckten Führungsbahn C durchstößt die Schwenkachse die sagittale Führungsebene E.

Das chirurgische Instrument 1 erlaubt eine Positionierung des Tibiaschnittblocks 700 relativ zur Referenzkomponente 600. Dabei kann eine sagittale Neigung des Tibiaschnittblocks 700 ohne ungewollte Beeinflussung einer sogenannten Tibiaschnitthöhe eingestellt werden. Die sagittale Neigung wird auch als Slope bezeichnet. Wünschenswerterweise erfolgt dabei die Positionierung des Tibiaschnittblocks 700 in Extension, d.h. bei gestrecktem Bein oder/und Kniegelenk. In Extension schließen der Femur F und die Tibia T einen Winkel von 180° ein. Mittels des chirurgischen Instruments 1 können dabei Abweichungen von der exakten Extensionsstellung bei der Einstellung des Slopes berücksichtigt werden. Somit lässt sich der Slope mittels des chirurgischen Instruments 1 auch dann präzise einstellen, wenn eine exakte Extensionsstellung nicht vorliegt.

Es ist ferner eine Fixiereinrichtung 303 vorhanden. Die Fixiereinrichtung 303 ist zur Fixierung der Beweglichkeit der Führungseinrichtung 300 eingerichtet. Somit lässt sich mittels der Fixiereinrichtung 303 die Winkelstellung zwischen der ersten und der zweiten Befestigungseinrichtung 100, 200 fixieren. Die Fixiereinrichtung 300 bewirkt eine lösbare kraftoder/und formschlüssige Verbindung zwischen der wenigstens einen Führungsstange 301 und der Führungsaufnahme 302. Beispielsweise kann die Fixiereinrichtung 303 ein Verspannen der Führungsstange 301 - vorliegend beider Führungsstangen 301 - bewirken. Zu diesem Zweck kann die Fixiereinrichtung 303 einen Schraub-, Klemm-, Rast- oder sonstigen Mechanismus aufweisen. Vorliegend ist die Fixiereinrichtung 302 mit einem Rastmechanismus ausgeführt. Bei einer Relativbewegung zwischen den Führungsstangen 301 und der Führungsaufnahme 302 wandert die Ablesemarke 901 entlang der Skale, insbesondere entlang der Teilung der Skale. Bei exakter Extension dient die Anzeigeeinrichtung 900 einer Anzeige der sagittalen Neigung, d.h. des Slope, des Tibiaschnittblocks 700.

Nachfolgend wird exemplarischen eine Verwendung des chirurgischen Instruments 1 zur Positionierung des Tibiaschnittblocks 700 erläutert.

Die Positionierung erfolgt ausgehend von einer Konfiguration, in welcher die Referenzkomponente 600 - beim vorliegend verfolgten all femur first Ansatz das femorale Probekondylenimplantat 601 - auf eine dem Fachmann bekannte Weise an dem distalen Femur F fixiert ist. Eine Ausrichtung des femoralen Probekondylenimplantats 601 lässt sich nun mittels des chirurgischen Instruments 1 auf den Tibiaschnittblock 700 transferieren.

Hierfür wird zunächst der Tibiaschnittblock 700 an der zweiten Befestigungseinrichtung 200 lösbar befestigt. Das chirurgische Instrument wird dann gemeinsam mit dem an demselben befestigten Tibiaschnittblock 700 femurseitig referenziert. Zu diesem Zweck wird die erste Befestigungseinrichtung 100 lösbar an dem Probekondylenimplantat 601 befestigt. Die lösbare Befestigung der ersten Befestigungseinrichtung an dem femoralen Probekondylenimplantat 601 kann mittels einer Steckverbindung realisiert sein. Auf diese Weise wird die in den Fig. 1 und 2 erkennbare Konfiguration erreicht. Sofern sich der Punkt, in welchem die Schwenkachse die sagittale Führungsebene E durchstößt, nicht an einer auf die Anatomie des Patienten bezogenen Sollposition befindet, lässt sich die Position mittels der ersten Linearführung 400 nachjustieren. Befindet sich der Punkt in seiner Sollposition, so wird der Ausrichtstab 800 entlang der anterioren Kante V der Tibia T ausgerichtet. Die Anzeigeeinrichtung 900 zeigt dann an, in welcher Winkelstellung sich der Ausrichtstab 800 und somit auch die zweite Befestigungseinrichtung 200 samt daran befestigtem Tibiaschnittblock 700 relativ zum femoralen Probekondylenimplantat 601 und damit relativ zur ersten Befestigungseinrichtung 100 befindet. Diese Winkelstellung repräsentiert einen vorherrschenden Beugungswinkel zwischen Femur F und Tibia T oder eine Abweichung dieses Winkels von der exakten Extension. Zur Einstellung des Slopes wird nun die zweite Befestigungseinrichtung 200 samt daran befestigtem Tibiaschnittblock 700 um genau den Winkelwert relativ zum Probekondylenimplantat 601 und relativ zur daran befestigten ersten Befestigungseinrichtung 100 geschwenkt, der dem Slope entspricht. Dieser Winkelwert des Slopes lässt sich ebenfalls von der Anzeigeeinrichtung 900 ablesen. Die Tibiaschnitthöhe wird hierbei nicht verändert, da wie beschrieben vorab die Positionierung der Schwenkachse erfolgt ist. Es versteht sich, dass der Ausrichtstab 800 in Zusammenwirkung mit der Anzeigeeinrichtung 900 auch dazu genutzt werden kann, Femur F und Tibia T in exakte Extension zu bringen.

## Patentansprüche

1. Chirurgisches Instrument (1) zur Verwendung bei einer Kniegelenkersatzoperation, aufweisend
eine erste Befestigungseinrichtung (100), welche zur lösbaren Befestigung an einer an einem distalen Femur (F) angebrachten Referenzkomponente (600) eingerichtet ist,
eine zweite Befestigungseinrichtung (200), welche distal von der ersten Befestigungseinrichtung (100) beabstandet und zur lösbaren Befestigung an einem Tibiaschnittblock (700) zur Schnittführung an einer proximalen Tibia (T) eingerichtet ist,
eine Führungseinrichtung (300), welche einenends mit der ersten Befestigungseinrichtung (100) und anderenends mit der zweiten Befestigungseinrichtung (200) verbunden ist und mittels welcher die zweite Befestigungseinrichtung (200) relativ zu der ersten Befestigungseinrichtung (100) in einer sagittalen Führungsebene (E) geführt schwenkbeweglich ist,
und einen Ausrichtstab (800), der zur Ausrichtung an einer anterioren Kante (V) der Tibia (T) eingerichtet ist, wobei der Ausrichtstab (800) mit der zweiten Befestigungseinrichtung (200) lösbar starr verbunden ist.

2. Chirurgisches Instrument (1) nach Anspruch 1, **dadurch gekennzeichnet, dass** die zweite Befestigungseinrichtung (200) einen Aufnahmeabschnitt (201) aufweist, in welchem ein Befestigungsabschnitt (801) des Ausrichtstabs (800) entlang einer Aufnahmerichtung (A) aufgenommen ist.

3. Chirurgisches Instrument (1) nach Anspruch 2, **dadurch gekennzeichnet, dass** der Befestigungsabschnitt (801) des Ausrichtstabs (800) wenigstens in einem Teilabschnitt (802) einen unrunden, insbesondere eckigen, Querschnitt aufweist, wobei der Aufnahmeabschnitt (201) der zweiten Befestigungseinrichtung (200) eine Formgebung aufweist, die zur lösbaren starren Verbindung des Ausrichtstabs (800) und der zweiten Befestigungseinrichtung (200) komplementär zu dem Befestigungsabschnitt (801), insbesondere wenigstens zu dem Teilabschnitt (802), des Ausrichtstabs (800) ausgebildet ist.

4. Chirurgisches Instrument (1) nach Anspruch 3, **dadurch gekennzeichnet, dass** der unrunde Querschnitt drehsymmetrisch, insbesondere punktsymmetrisch, ausgebildet ist, so dass der Ausrichtstab (800) zur lösbaren starren Verbindung mit der zweiten Befestigungseinrichtung (200) in wenigstens zwei Winkelpositionen relativ zur zweiten Befestigungseinrichtung (200) in dem Aufnahmeabschnitt (201) aufnehmbar ist.

5. Chirurgisches Instrument (1) nach einem der vorhergehenden Ansprüche, **dadurch gekennzeichnet, dass** der Ausrichtstab (800) S-förmig gebogen ist.

6. Chirurgisches Instrument (1) nach einem der vorhergehenden Ansprüche, **dadurch gekennzeichnet, dass** die zweite Befestigungseinrichtung (200) einen Handhabungsabschnitt (203) aufweist, dessen Formgebung auf eine Anatomie einer Hand eines Benutzers abgestimmt ist.

7. Chirurgisches Instrument (1) nach Anspruch 6, **dadurch gekennzeichnet, dass** die zweite Befestigungseinrichtung (200) einen, insbesondere plattenförmigen, Grundkörper (202) umfasst, an welchem zur Ausbildung des Handhabungsabschnitts (203) eine Ausnehmung (204) vorhanden ist, die zum Aufnehmen wenigstens eines Fingers der Hand des Benutzers ausgebildet ist.

8. Chirurgisches Instrument (1) nach einem der vorhergehenden Ansprüche, **dadurch gekennzeichnet, dass** eine Anzeigeeinrichtung (900) vorhanden ist, die zum Anzeigen einer Winkelstellung der zweiten Befestigungseinrichtung (200) in der Führungsebene (E) relativ zur ersten Befestigungseinrichtung (100) eingerichtet ist.

9. Chirurgisches Instrument (1) nach Anspruch 8, **dadurch gekennzeichnet, dass** die Anzeigeeinrichtung (900) wenigstens eine Skale und wenigstens eine Ablesemarke (901) umfasst, wobei die Skale mit der ersten Befestigungseinrichtung (100) und die Ablesemarke (901) mit der Führungseinrichtung (300) verbunden ist oder umgekehrt, und wobei eine Position der Ablesemarke (901) relativ zur Skale die momentaneWinkelstellung der zweiten Befestigungseinrichtung (200) in der Führungsebene (E) relativ zur ersten Befestigungseinrichtung (100) repräsentiert.

10. Chirurgisches Instrument (1) nach einem der vorhergehenden Ansprüche, **dadurch gekennzeichnet, dass** die Führungseinrichtung (300) wenigstens eine gebogene Führungsstange (301) und eine Führungsaufnahme (302) aufweist, in welcher die Führungsstange (301) gleitbeweglich geführt aufgenommen ist, wobei die Führungsaufnahme (302) mit der ersten Befestigungseinrichtung (100) und die Führungsstange (301) mit der zweiten Befestigungseinrichtung (200) verbunden ist oder umgekehrt, und wobei die zweite Befestigungseinrichtung (200) mittels der Führungseinrichtung (300) entlang einer kreisbogenförmig längserstreckten Führungsbahn (C) relativ zur ersten Befestigungseinrichtung (100) linearbeweglich geführt ist.

11. Chirurgisches Instrument (1) nach einem der vorhergehenden Ansprüche, **dadurch gekennzeichnet, dass** eine erste Linearführung (400) vorhanden ist, mittels derer die Führungseinrichtung (300) mit der ersten Befestigungseinrichtung (100) verbunden und relativ zu derselben in der Führungsebene (E) entlang einer anteroposterior längserstreckten ersten Führungsbahn (L1) geführt linearbeweglich ist.

12. Chirurgisches Instrument (1) nach einem der vorhergehenden Ansprüche, **dadurch gekennzeichnet, dass** eine zweite Linearführung (500) vorhanden ist, mittels derer die zweite Befestigungseinrichtung (200) mit der Führungseinrichtung (300) verbunden und relativ zu derselben in der Führungsebene (E) entlang einer anteroposterior längserstreckten zweiten Führungsbahn (L2) geführt linearbeweglich ist.

13. Chirurgisches Instrument (1) nach einem der vorhergehenden Ansprüche, **dadurch gekennzeichnet, dass** eine Fixiereinrichtung (303) vorhanden und zur Fixierung der Beweglichkeit der Führungseinrichtung (300) eingerichtet ist.

14. Chirurgisches Instrumentensystem (10) mit einem chirurgischen Instrument (1) nach einem der vorhergehenden Ansprüche, einer Referenzkomponente (600), die lösbar an der ersten Befestigungseinrichtung (100) des chirurgischen Instruments (1) befestigt ist, und mit einem Tibiaschnittblock (700), der lösbar an der zweiten Befestigungseinrichtung (200) des chirurgischen Instruments (1) befestigt ist.

## Claims

1. Surgical instrument (1) for use in a knee joint replacement operation having
a first fastening device (100) which is configured for releasable fastening to a reference component (600) which is fitted to a distal femur (F),
a second fastening device (200) which is distally spaced apart from the first fastening device (100) and which is configured for releasable fastening to a tibia cutting block (700) for guiding cutting on a proximal tibia (T),
a guiding device (300) which is connected at one end to the first fastening device (100) and at the other end to the second fastening device (200) and by means of which the second fastening device (200) is pivotably movable in a manner guided relative to the first fastening device (100) in a sagittal guiding plane (E),
and an alignment rod (800) which is configured for alignment with respect to an anterior edge (V) of the tibia (T), wherein the alignment rod (800) is releasably connected to the second fastening device (200) in a rigid manner.

2. Surgical instrument (1) according to claim 1, **characterised in that** the second fastening device (200) has a receiving portion (201) in which a fastening portion (801) of the alignment rod (800) is received in a receiving direction (A).

3. Surgical instrument (1) according to claim 2, **characterised in that** the fastening portion (801) of the alignment rod (800) has at least in a part-portion (802) a non-round, particularly angular cross section, wherein the receiving portion (201) of the second fastening device (200) has a shape which is constructed for releasable rigid connection of the alignment rod (800) and the second fastening device (200) so as to complement the fastening portion (801), in particular at least the part-portion (802) of the alignment rod (800).

4. Surgical instrument (1) according to claim 3, **characterised in that** the non-round cross section is constructed in a rotationally symmetrical, in particular point-symmetrical manner, so that the alignment rod (800) can be received for releasable rigid connection to the second fastening device (200) in at least two angular positions relative to the second fastening device (200) in the receiving portion (201).

5. Surgical instrument (1) according to any one of the preceding claims, **characterised in that** the alignment rod (800) is bent in an S-shaped manner.

6. Surgical instrument (1) according to any one of the preceding claims, **characterised in that** the second fastening device (200) has a handling portion (203), the shape of which is adapted to an anatomy of a hand of a user.

7. Surgical instrument (1) according to claim 6, **characterised in that** the second fastening device (200) comprises a, in particular plate-like, base member (202) on which in order to form the handling portion (203) there is provided a recess (204) which is constructed to receive at least one finger of the hand of the user.

8. Surgical instrument (1) according to any one of the preceding claims, **characterised in that** there is provided a display device (900) which is configured to display an angular position of the second fastening device (200) in the guiding plane (E) relative to the first fastening device (100).

9. Surgical instrument (1) according to claim 8, **characterised in that** the display device (900) comprises at least one scale and at least one reading mark (901), wherein the scale is connected to the first fastening device (100) and the reading mark (901) is connected to the guiding device (300), or vice versa, and wherein a position of the reading mark (901) relative to the scale represents the current angular position of the second fastening device (200) in the guiding plane (E) relative to the first fastening device (100).

10. Surgical instrument (1) according to any one of the preceding claims, **characterised in that** the guiding device (300) has at least one curved guiding rod (301) and a guide receiving member (302) in which the guiding rod (301) is received in a state guided in a slidingly movable manner, wherein the guide receiving member (302) is connected to the first fastening device (100) and the guiding rod (301) is connected to the second fastening device (200), or vice versa, and wherein the second fastening device (200) is guided in a linearly movable manner by means of the guiding device (300) along a guiding path (C) which is elongate in a circular-arc-like manner relative to the first fastening device (100).

11. Surgical instrument (1) according to any one of the preceding claims, **characterised in that** there is provided a first linear guide (400), by means of which the guiding device (300) is connected to the first fastening device (100) and is linearly movable in a manner guided relative thereto in the guiding plane (E) along a first guiding path (L1) which is elongate in an anteroposterior manner.

12. Surgical instrument (1) according to any one of the preceding claims, **characterised in that** there is provided a second linear guide (500), by means of which the second fastening device (200) is connected to the guiding device (300) and is linearly movable in a manner guided relative thereto in the guiding plane (E) along a second guiding path (L2) which is elongate in an anteroposterior manner.

13. Surgical instrument (1) according to any one of the preceding claims, **characterised in that** a fixing device (303) is provided and is configured to fix the movability of the guiding device (300).

14. Surgical instrument system (10) having a surgical instrument (1) according to any one of the preceding claims, a reference component (600) which is releasably fastened to the first fastening device (100) of the surgical instrument (1), and having a tibia cutting block (700) which is releasably fastened to the second fastening device (200) of the surgical instrument (1).

## Revendications

1. Instrument chirurgical (1) destiné à être utilisé lors d'une opération de remplacement de l'articulation du genou, présentant
un premier dispositif de fixation (100) qui est conçu pour la fixation amovible à un composant de référence (600) appliqué sur un fémur distal (F),
un second dispositif de fixation (200), qui est écarté de manière distale du premier dispositif de fixation (100) et qui est conçu pour la fixation amovible à un bloc de coupe tibial (700) pour le guidage de la coupe au niveau d'un tibia proximal (T),
un dispositif de guidage (300) qui est relié en une extrémité au premier dispositif de fixation (100) et en l'autre extrémité au second dispositif de fixation (200) et au moyen duquel le second dispositif de fixation (200) peut pivoter de manière guidée par rapport au premier dispositif de fixation (100) dans un plan de guidage sagittal (E),
et une tige d'orientation (800) qui est conçue pour l'orientation au niveau d'un bord antérieur (V) du tibia (T), la tige d'orientation (800) étant reliée de manière rigide et amovible au second dispositif de fixation (200).

2. Instrument chirurgical (1) selon la revendication 1, **caractérisé en ce que** le second dispositif de fixation (200) présente une section de logement (201) dans laquelle est logée une section de fixation (801) de la tige d'orientation (800) le long d'un sens de logement (A).

3. Instrument chirurgical (1) selon la revendication 2, **caractérisé en ce que** la section de fixation (801) de la tige d'orientation (800) présente, au moins dans une section partielle (802), une section transversale non ronde, en particulier rectangulaire, la section de logement (201) du second dispositif de fixation (200) présentant une forme qui est conçue, pour la liaison rigide et amovible de la tige d'orientation (800) et du second dispositif de fixation (200), de manière complémentaire à la section de fixation (801), en particulier au moins à la section partielle (802), de la tige d'orientation (800).

4. Instrument chirurgical (1) selon la revendication 3, **caractérisé en ce que** la section transversale non ronde est conçue avec une symétrie de rotation, en particulier une symétrie ponctuelle, de telle sorte que la tige d'orientation (800), pour la liaison rigide et amovible au second dispositif de fixation (200), peut être logée dans la section de logement (201) dans au moins deux positions angulaires par rapport au second dispositif de fixation (200).

5. Instrument chirurgical (1) selon l'une des revendications précédentes, **caractérisé en ce que** la tige d'orientation (800) est courbée en forme de S.

6. Instrument chirurgical (1) selon l'une des revendications précédentes, **caractérisé en ce que** le second dispositif de fixation (200) présente une section de manipulation (203) dont la forme est adaptée à l'anatomie d'une main d'un utilisateur.

7. Instrument chirurgical (1) selon la revendication 6, **caractérisé en ce que** le second dispositif de fixation (200) comprend un corps de base (202), en particulier en forme de plaque, au niveau duquel se trouve un évidement (204) destiné à former la section de manipulation (203), lequel évidement est conçu pour le logement d'au moins un doigt de la main de l'utilisateur.

8. Instrument chirurgical (1) selon l'une des revendications précédentes, **caractérisé en ce qu'**un dispositif indicateur (900) est présent qui est conçu pour indiquer une position angulaire du second dispositif de fixation (200) par rapport au premier dispositif de fixation (100) dans le plan de guidage (E).

9. Instrument chirurgical (1) selon la revendication 8, **caractérisé en ce que** le dispositif indicateur (900) comprend au moins une échelle et au moins une marque de lecture (901), l'échelle étant reliée au premier dispositif de fixation (100) et la marque de lecture (901) étant reliée au dispositif de guidage (300) ou inversement et une position de la marque de lecture (901) par rapport à l'échelle représentant la position angulaire momentanée du second dispositif de fixation (200) par rapport au premier dispositif de fixation (100) dans le plan de guidage (E).

10. Instrument chirurgical (1) selon l'une des revendications précédentes, **caractérisé en ce que** le dispositif de guidage (300) présente au moins une tige de guidage (301) incurvée et un logement de guidage (302) dans lequel la tige de guidage (301) est logée de manière guidée mobile en coulissement, le logement de guidage (302) étant relié au premier dispositif de fixation (100) et la tige de guidage (301) étant reliée au second dispositif de fixation (200) ou inversement et le second dispositif de fixation (200) étant guidé de manière linéairement mobile, au moyen du dispositif de guidage (300), par rapport au premier dispositif de fixation (100) le long d'une voie de guidage (C) s'étendant longitudinalement en forme d'arc de cercle.

11. Instrument chirurgical (1) selon l'une des revendications précédentes, **caractérisé en ce qu'**un premier guidage linéaire (400) est présent, au moyen duquel le dispositif de guidage (300) est relié au premier dispositif de fixation (100) et guidé de manière linéairement mobile par rapport à celui-ci dans le plan de guidage (E) le long d'une première voie de guidage (L1) s'étendant longitudinalement de manière antéropostérieure.

12. Instrument chirurgical (1) selon l'une des revendications précédentes, **caractérisé en ce qu'**un second guidage linéaire (500) est présent, au moyen duquel le second dispositif de fixation (200) est relié au dispositif de guidage (300) et guidé de manière linéairement mobile par rapport à celui-ci dans le plan de guidage (E) le long d'une seconde voie de guidage (L2) s'étendant longitudinalement de manière antéropostérieure.

13. Instrument chirurgical (1) selon l'une des revendications précédentes, **caractérisé en ce qu'**un dispositif de fixation (303) est présent et conçu pour la fixation de la mobilité du dispositif de guidage (300).

14. Système d'instrument chirurgical (10) présentant un instrument chirurgical (1) selon l'une des revendications précédentes, un composant de référence (600), qui est fixé de manière amovible au premier dispositif de fixation (100) de l'instrument chirurgical (1), et présentant un bloc de coupe tibial (700), qui est fixé de manière amovible au second dispositif de fixation (200) de l'instrument chirurgical (1).
